# EUROPEAN PATENT APPLICATION

(11) **EP 4 302 594 A1**
(43) Date of publication of application: **10.01.2024**
(21) Application number: 22853366.7
(22) Date of filing: 29.07.2022
(51) Int. Cl.: A01K 1/00, A61L 2/06, A01K 31/00, A01K 31/22, A61L 9/16

(54) **DISEASE PREVENTION SYSTEM USING HOT AIR FOR POULTRY BARN HAVING MULTI-ROW MULTI-LAYERED CAGE**

(30) Priority: 03.08.2021 KR 20210102146
(71) Applicant: Safek Inc., Jeju-si, Jeju-do 63110 (KR)
(72) Inventor: MOON, Chang Sub, Seogwipo-si, Jeju-do 63527 (KR)
(74) Representative: Caspary, Karsten
(86) International application number: PCT/KR2022/011184
(87) International publication number: WO 2023/013988

(57) **Abstract**

The present disclosure relates to a hot-air disinfection system for supplying and circulating a hot air throughout an interior of a poultry barn having multi-row multi-layered cages to uniformly maintain an internal temperature of the poultry barn and enhance disinfection efficiency, which includes: a heat source supply part configured to supply the hot air to the interior of the poultry barn; a heat distributer connected to the heat source supply part to distribute the hot air supplied thereto; a plurality of horizontal heat transfer pipes connected to the heat distributer to transfer the hot air supplied from the heat distributer; an vertical heat blower pipe provided vertically in the interior of the poultry barn to blow the hot air near a ceiling of the barn toward the floor; and a control part configured to control an operation of the heat source supply part.

## Description

### FIELD

The present disclosure relates to a hot-air disinfection system for a poultry barn having multi-row and multi-level cages, and more particularly, to a hot-air disinfection system for a poultry barn having multi-row and multi-level cages, in which hot air can be supplied and circulated throughout the poultry barn having the multi-row and multi-level cages so that an internal temperature of the barn is uniformly kept, and thus improving a disinfection efficiency.

### BACKGROUND

The modern stock-raising industry has a property in which breeding is performed in a close manner in a window-free barn, which is a large space.

A layer chicken or window-free poultry barn has a function of suppressing bacteria, viruses, and pests from entering the interior of the barn from the outside, and also a function of preventing such bacteria, viruses, and pests that entered the barn from being exposed to external ultraviolet rays and defending against natural enemies, or competing organisms. This causes various infectious diseases.

Heat-based sterilization and insecticide is eco-friendly due to the high development of modern science, which is known as safe sterilization and insecticide methods.

Disinfection using hot air utilizes the principle that proteins coagulate due to heat. In general, the proteins begin to coagulate at temperatures above 40 degrees, and most proteins coagulate at around 60 degrees.

Therefore, such a disinfection method is widely used because it is known to be highly effective against bacteria, viruses, and pests. However, the method is difficult to control a heat source supplied from the inside or outside. Since the hot air is concentrated near a ceiling of the barn, structures vulnerable to heat, such as various batteries, electronic components, and PE wires installed in the poultry barn having the multi-row and multi-level cage may be damaged. In addition, there is a high risk of fire.

In addition, the heat source is limited to some areas, or a temperature near the ceiling is high while a temperature near the floor is below 40 degrees. This reduces disinfection efficiency and reduces energy efficiency due to heat loss.

Therefore, the method is being shunned by agricultural, livestock and fishery businesses due to the risk of fire by the hot air, loss of various equipment, and low heat efficiency.

In addition, a representative barn for raising layer chickens or poultries is a large structure with a length of 102 meters, an internal width of 11 meters, and a height of 8.8 meters. In the structure, an iron-made cage of 4 rows and 8 stages with a width of 1.5 meters is provided at a length of 96 meters, which greatly impedes the circulation of the heat source. Due to such matters, in the case of the layer chicken or poultry cage described above, the supplied heat source stagnates within a distance of 10 meters in a longitudinal direction of the cage at the time of supply of the heat source. This may cause a risk of fire due to high temperature. The temperature is uneven throughout an internal space of the barn, which greatly reduces the disinfection efficiency.

The present disclosure is proposed to solve the aforementioned matters, and the present disclosure is for the purpose of providing a hot-air disinfection system for a poultry barn having multi-row and multi-level cages in which at least one heat source supply part configured to supply a heat source to the interior of the poultry barn having multi-row multi-layer cages is provided to eliminate the need to install a heat distributor, a temperature of hot air, which is capable of reducing installation costs and maximizing disinfection and energy efficiency by recirculating air so that a temperature of hot air is maintained uniformly throughout a ceiling, floor, and front and rear portions of the barn.

### SUMMARY

A hot-air disinfection system for a poultry barn having multi-row and multi-level cages according to an example embodiment of the present disclosure for accomplishing the above-mentioned objectives may include: a heat source supply part configured to supply a hot air to an interior of the poultry barn having the multi-row and multi-level cages; a plurality of horizontal heat transfer pipes which are connected to or provided respectively between an outer wall of the poultry barn and the multi-row and multi-level cage or between the multi-row and multi-level cages while being spaced apart from the heat source supply part so that the hot air supplied from the heat source supply part is transferred to a floor of the poultry barn, ambient air and the multi-row and multi-level cages. The plurality of horizontal heat transfer pipes, both ends of which are open, are configured to transfer the hot air; a vertical heat blower pipe, both ends of which are open, provided vertically in the interior of the poultry barn and configured to blow the hot air near a ceiling of the poultry barn toward the floor by a blower fan provided inside the vertical heat blower pipe; and a control part configured to control an operation of the heat source supply part.

The hot-air disinfection system may further include an air circulation pipe connected between the heat source supply part and the poultry barn and configured to circulate air in the interior of the poultry barn.

The air circulation pipe may include a filter configured to filter floating matters in the interior of the poultry barn.

The heat source supply part may be provided outside the poultry barn and connected to a heat distributer via a front entrance or a rear entrance of the poultry barn including a feed bin and an air suction window so as to supply the hot air to the heat distributer.

The heat source supply part may include at least one or more heat source supply parts provided in passages between the multi-row and multi-level cages in the interior of the poultry barn.

A flow rate of the hot air supplied from the heat source supply part may be 5.5 to 7.2 times a total volume of the poultry barn per hour.

The heat distributor may distribute and supply the hot air supplied from the heat source supply part to each of the plurality of horizontal heat transfer pipes, and a temperature of air at an outlet connected to each of the plurality of horizontal heat transfer pipes may be in a range of 60 to 80 degrees CC.

The plurality of horizontal heat transfer pipe may be connected to the heat source supply part or the heat distributor and provided respectively between the outer wall of the poultry barn and the multi-row and multi-level cages or spaces between the multiseriate multi-layered cages, so that the hot air supplied from the heat source supply part is transferred to the floor of the poultry barn, air around the plurality of horizontal heat transfer pipe and the multi-row and multi-level cages.

When the plurality of horizontal heat transfer pipes are not directly connected to the heat source supply part or the heat distributer, the blower fan may be provided inside each of the plurality of horizontal heat transfer pipes to transfer the hot air.

A temperature of the hot air supplied from the heat source supply part to the plurality of horizontal heat transfer pipes in an initial stage may be in a range of 50 to 70 degrees C, and a temperature of the hot air at an end of each of the plurality of horizontal heat transfer pipes may be in a range of 45 to 60 degrees C.

A length of each of the plurality of horizontal heat transfer pipes may be adjusted according to a position at which the heat source supply part is arranged.

In a case where the poultry barn has not a layered structure, the vertical heat blower pipe may be provided between the multi-row and multi-level cages, while in a case where the poultry barn has not a layered structure, the vertical heat blower pipe may be provided in an internal space of each of the multi-row and multi-level cages such that the vertical heat blower pipe extends near the floor and comes out of a space between the multi-row and multi-level cages near the floor so as to blow the hot air around the ceiling downward.

An upper end portion of the vertical heat blower pipe may be located higher than an upper end of each of the multi-row and multi-level cages and provided below a support structure at a position of at least 85% or higher of a height from the floor of the poultry barn to the support structure.

The upper end portion of the vertical heat blower pipe may be located at a place spaced apart by a distance of 1.5 to 3 meters from the floor of the poultry barn.

The vertical heat blower pipe may include at least one or more vertical heat blower pipes provided in a rear portion opposite an entrance of the poultry barn.

The vertical heat blower pipe, which is provided vertically in the poultry barn, may extend to the floor of the poultry barn and may be directly connected to a rear end of the poultry barn so as to implement both a vertical heat transfer and a horizontal heat transfer.

The vertical heat blower pipe may be connected to or provided spaced apart from the plurality of horizontal heat transfer pipes according to a blowing amount.

A temperature sensor may be provided in the ceiling of the poultry barn. When a temperature of the ceiling is 70 degrees C or higher, the operation of the heat source supply part may be stopped to circulate air in an air circulation pipe under the control of the control part.

Silica (silicon dioxide) may be sprayed onto a wall or the floor of the poultry barn to eliminate mites by a dry phenomenon before disinfection using the hot air.

As described above, with a hot-air disinfection system for a poultry barn having multi-row and multi-level cages according to the present disclosure, by transferring the hot air, which is generated from a heat source supply part provided outside or inside the barn, from a front entrance to a rear portion of the barn through a horizontal heat transfer pipe provided between an outer wall of the barn and the cage or between adjacent cages, connecting a vertical heat transfer pipe to the horizontal heat transfer pipe up to the vicinity of a ceiling in a vertical direction, and blowing the air in the vicinity of the ceiling downward, it is possible to improve the efficiency of the supply of the hot air and blow high-temperature heat in the vicinity of the ceiling downward, thus keeping the temperature throughout the barn as constant as possible and maximizing the disinfection efficiency.

### BRIEF DESCRIPTION OF THE DRAWINGS

FIG. 1 is a configuration diagram illustrating a hot-air disinfection system for a poultry barn having multi-row and multi-level cages according to the present disclosure.
FIG. 2 is a perspective view illustrating a barn having a hot-air disinfection system for poultry barn having a multi-row and multi-level cages according to the present disclosure.
FIG. 3 is a front view illustrating an interior of a barn including the hot-air disinfection system for the poultry barn having the multi-row multi-layer cages according to the present disclosure.
FIG. 4 is a side view illustrating the interior of the barn including the hot-air disinfection system for the poultry barn having the multi-row multi-layer cage according to the present disclosure.
FIG. 5 is a plan view illustrating the interior of the barn including the hot-air disinfection system for the poultry barn having the multi-row multi-layer cage according to the present disclosure.
FIG. 6 is a perspective view illustrating an example of the barn including the hot-air disinfection system for the poultry barn having the multi-row and multi-level cage according to the present disclosure.
FIG. 7 is a cross-sectional view illustrating a state where a vertical heat blower pipe and a horizontal heat transfer pipe are connected to a heat source supply part or a heat distributer in the hot-air disinfection system for the poultry barn having the multi-row and multi-level cages according to the present disclosure.
FIG. 8 is a front view illustrating a state where the vertical heat blower pipe and the horizontal heat transfer pipe are provided to be spaced apart from the heat source supply part or the heat distributer in the hot-air disinfection system for the poultry barn having the multi-row and multi-level cages according to the present disclosure.
FIG. 9 is a side view illustrating another example of the hot-air disinfection system for the poultry barn having the multi-row and multi-level cages according to the present disclosure.
FIG. 10 is a side view illustrating still another example of the hot-air disinfection system for the poultry barn having the multi-row and multi-level cages according to the present disclosure.

### EXPLANATION OF REFERENCE NUMERALS

| | | | |
|---|---|---|---|
| 10: | Barn | 11: | Support structure |
| 20: | Cage | 100: | Heat source supply part |
| 110: | Air circulation pipe | 120: | Filter |
| 200: | Heat distributor | 300: | Horizontal heat transfer pipe |
| 400: | Vertical heat blower pipe | 500: | Controller |
| 600: | Temperature sensor | F: | Blower fan |

### DETAILED DESCRIPTION

Various modifications may be made to the present disclosure as various example embodiments and specific example embodiments will be described in detail in the description with reference to the drawings. However, the present disclosure is not limited to such specific example embodiments and should be understood as including all modifications, equivalents, alternatives falling within the spirit and scope of the present disclosure.

The example embodiments of the present disclosure are not limited to specific forms illustrated in the drawings, and each drawing may be illustrated on a large scale for clarification. Specific terms have been used in this specification, they are merely specific examples disclosed to easily explain the present disclosure and are not intended to limit the scope of the present disclosure recited in the claims.

Further, the expression "and/or" used herein may be used to include at least one of constituent elements arranged in forward and backward directions. Further, the expression "connected/coupled" may be used to include that a constituent element is directly connected or coupled to another constituent element, or is connected or coupled to another constituent element by intervening yet another constituent element therebetween. Further, the singular form described herein may include the plural form unless the context clearly dictates otherwise. The expression "comprises", "includes" or the like are used to include that a constituent element, a step, an operation and an element further comprises or includes other constituent elements, other steps, other operations and other elements.

In the description of various example embodiments, the description that a layer (film), region, pattern, or structure is formed "on" or "under" a substrate, side (film), region, pad, or pattern should be understood to encompass that the layer (film), region, pattern, or structure is directly formed on (or under) the substrate, side (film), region, pad, or pattern, or by intervening another layer therebetween. The standards relating to "on/above or below/under each layer" will be described with reference to the drawings.

Hereinafter, example embodiments of the present disclosure will be described in detail with reference to the accompanying drawings.

The poultry barn having multi-row and multi-level cages to be applied of the present disclosure is targeted to the most currently used barn as one example. Specifically, the targeted barn has a structure in which a length is 102 meters, an internal width is 11 meters, a height from a floor to a support structure located above the cage is 7 meters, a height from the floor to the top of a roof is 8.8 meters, and an iron-made cage of a width of 1.5 meters is arranged in 4 rows and 8 stages. However, the present disclosure is limited thereto and is applicable to a poultry barn having multi-row and multi-level cages similar to the above-described barn.

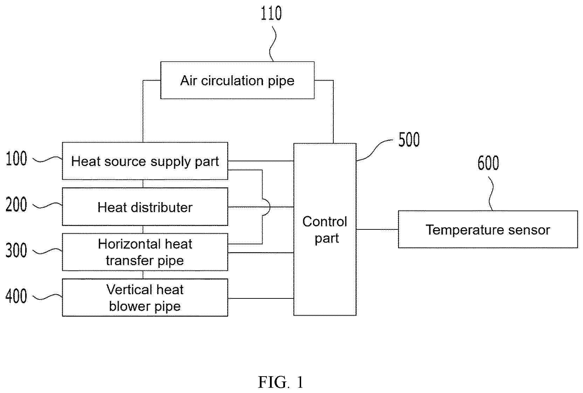

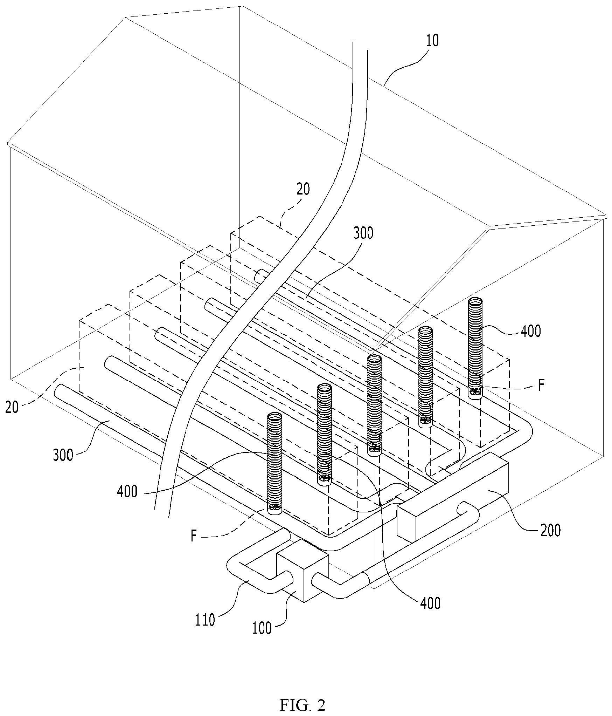

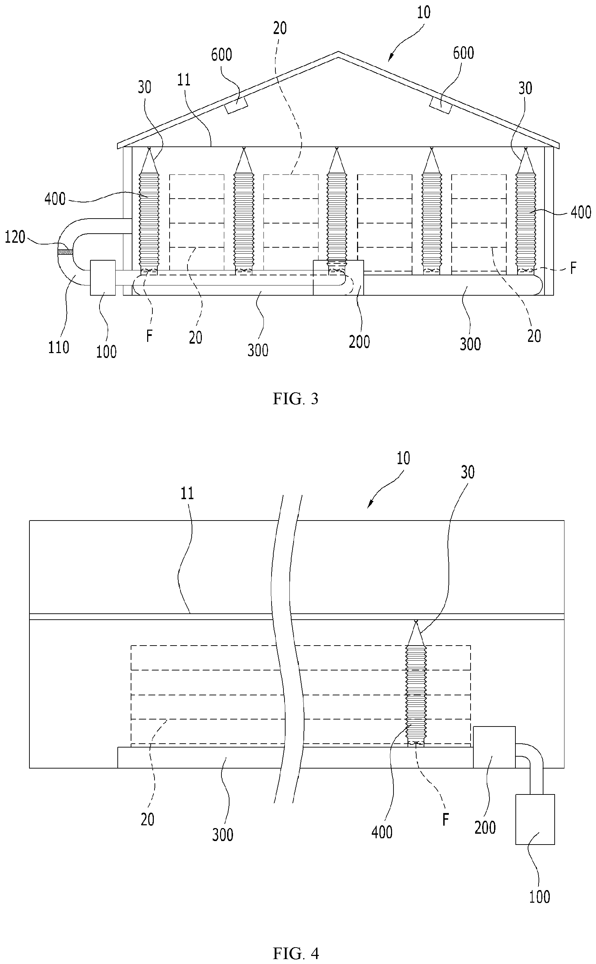

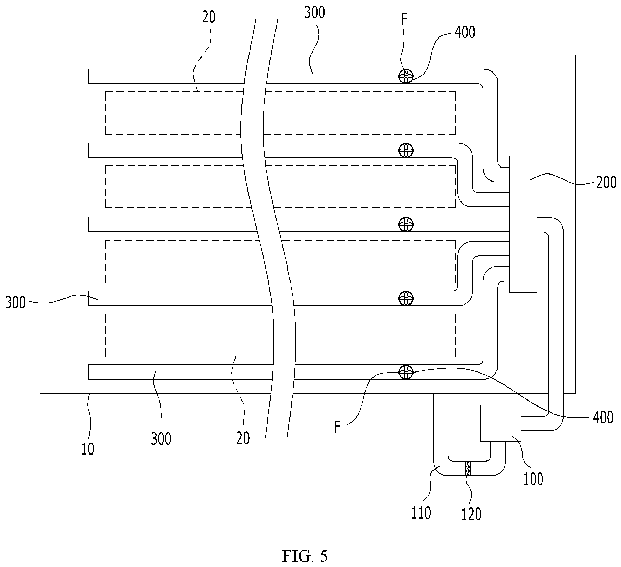

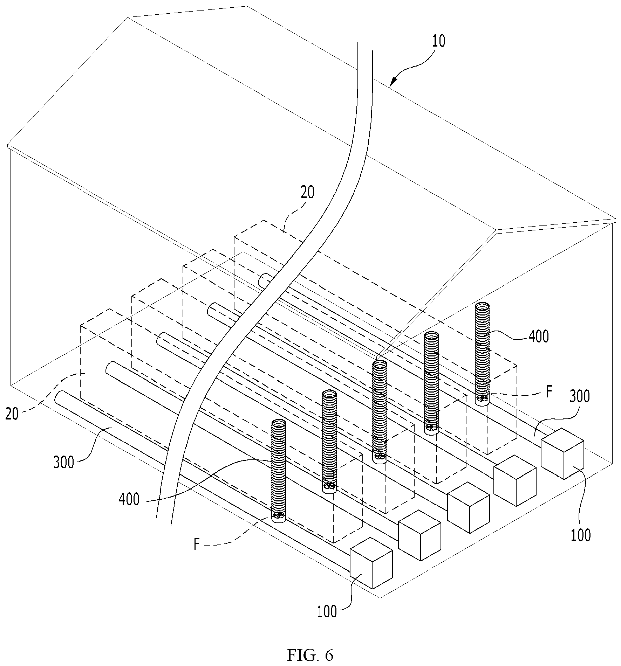

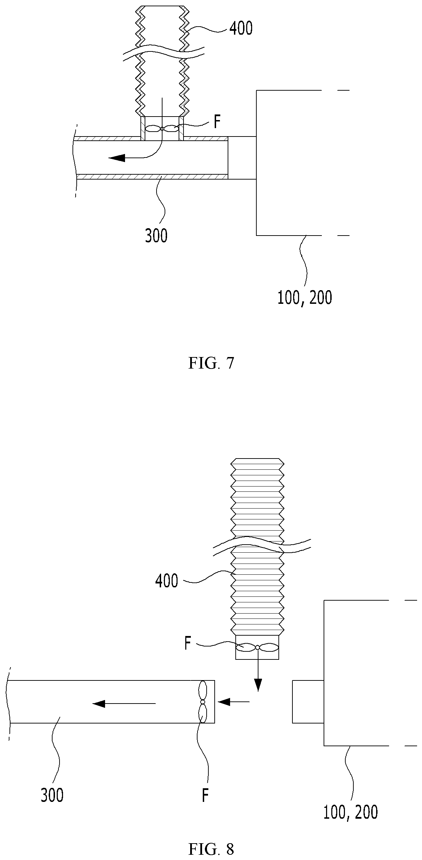

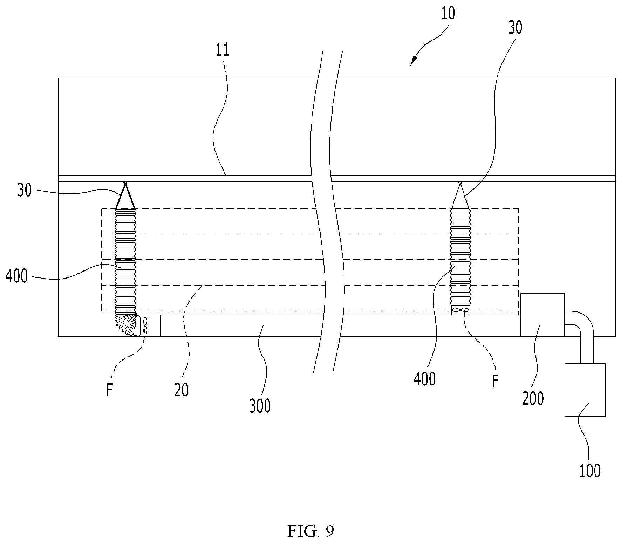

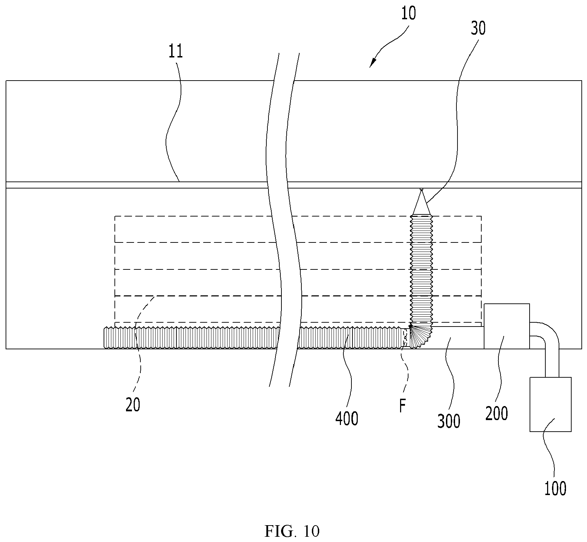

FIG. 1 is a configuration diagram illustrating a hot-air disinfection system for a poultry barn having a multi-row and multi-level cages according to the present disclosure. FIG. 2 is a perspective view illustrating a barn having a hot-air disinfection system for poultry barn having a multi-row and multi-levels cage according to the present disclosure. FIG. 3 is a front view illustrating an interior of a barn including the hot-air disinfection system for the poultry barn having the multi-row and multi-level cages according to the present disclosure. FIG. 4 is a side view illustrating the interior of the barn including the hot-air disinfection system for the poultry barn having the multi-row and multi-level cages according to the present disclosure. FIG. 5 is a plan view illustrating the interior of the barn including the hot-air disinfection system for the poultry barn having the multi-row and multi-level cages according to the present disclosure. FIG. 6 is a perspective view illustrating an example of the barn including the hot-air disinfection system for the poultry barn having the multi-row and multi-level cages according to the present disclosure. FIG. 7 is a cross-sectional view illustrating a state where a vertical heat blower pipe and a horizontal heat transfer pipe are connected to a heat source supply part or a heat distributer in the hot-air disinfection system for the poultry barn having the multi-row and multi-level cage according to the present disclosure. FIG. 8 is a front view illustrating a state where the vertical heat blower pipe and the horizontal heat transfer pipe are provided to be spaced apart from the heat source supply part or the heat distributer in the hot-air disinfection system for the poultry barn having the multi-row and multi-level cage according to the present disclosure. FIG. 9 is a side view illustrating another example of the hot-air disinfection system for the poultry barn having the multi-row and multi-level cage according to the present disclosure. FIG. 10 is a side view illustrating still another example of the hot-air disinfection system for the poultry barn having the multi-row and multi-level cage according to the present disclosure.

As illustrated in FIGS. 1 to 10, the hot-air disinfection system for the poultry barn having the multi-row and multi-level cage according to the present disclosure may include a heat source supply part 100, a heat distributer 200, a horizontal heat transfer pipe 300, a vertical heat blower pipe 400, and a control part 500.

The heat source supply part 100 supplies hot air to an interior of a barn 10. As the heat source supply part 100, a burner using various raw materials such as electricity, wood, and petroleum may be used. Preferably, a petroleum burner may be used.

In addition, the heat source supply part 100 may supply the hot air of 100,000 kcal~ 200,000 kcal per hour at the start of operation with the burner. When an internal temperature of the barn reaches a target temperature as the temperature increases, the heat source supply part 100 reduces an amount of the hot air to be supplied. Once the internal temperature of the barn is checked to reach the target temperature by the temperature sensor 600, the heat source supply part 100 stops the supply of the hot air and supplies the heat source according to a control signal from the control part 500. In this case, a capacity of the burner may be a level of 100,000 kcal per hour. A plurality of burners having a small capacity may be provided. The amount of the hot air supplied from the heat source supply part 100 is preferably 48,000 m'/h to 64,000 m'/h. An operation time may be adjusted taking into account season, weather, close property, and the like. In addition, such hot air may preferably by supplied using one or more burners. In other words, the flow rate of the hot air to be supplied corresponds to 5.5 to 7.2 times the total volume of the barn per hour. An amount of heat energy supplied per unit cubic meter of the barn for one hour may preferably by 13 to 26 kcal/(m³h) at an initial operation.

That is, as an example, in a case of using two burners having a capacity of 100,000 kcal per hour, the amount and temperature of the hot air, which are obtained by one burner of the above-described heat source supply part 100, are 5,000 m³/h and 150 degrees C itself. In a case where the hot air forcibly mixes three times the internal air of the barn 10 to generate an air volume of 20,000 m³/h, the hot air having a temperature of 60 to 80 degrees C may be obtained. In the case where the two burners are used as the heat source supply part 100, the hot air of about 40,000 m³/h may be obtained.

The heat source supply part 100 may be provided outside or inside the barn 10, and may supply the hot air via a feed bin and a front outlet or a rear outlet of the barn 10 including an air suction window. In this case, the heat source supply part 100 is preferably provided outside the barn 10 in consideration of fire risk due to high heat.

In addition, as illustrated in FIG. 6, at least one or more heat source supply parts 100 may be provided in passages between adjacent layer chicken cages 20 inside the barn 10. For a normal barn, four to six heat source supply parts 100 may be provided.

Further, an air circulation pipe 110 may be connected between the heat source supply part 100 and the barn 10 to reheat and circulate the internal air of the barn 10, thus increasing energy efficiency.

The air circulation pipe 110 may be provided with a filter 120 to suppress and filter soot from being generated when floating substances and the like in the interior of the barn are burned and carbonized in the heat source supply part 100 due to continuously-reused air.

In an example embodiment, when a petroleum burner is used as the heat source supply part 100, a temperature of an iron plate constituting a discharge port of the petroleum burner is 200 to 300 degrees C, and when an electric hot air is used as the heat source supply part 100, the temperature thereof is 150 degrees C or higher. A temperature of air discharged initially from the discharge port of the heat source supply part 100 is 50 to 200 degrees C, preferably 50 to 80 degrees C. When the temperature is more than 200 degrees C, there is a risk of fire.

In addition, since the heat source discharged from the heat source supply part 100 has a temperature of 100 degrees C or higher, it is desirable to supply the hot air into the barn 10 via an insulation pipe.

The heat distributor 200 may be connected to the heat source supply part 100 and connects horizontal heat transfer pipes 300 to each other so that the hot air supplied from the heat source supply part 100 is supplied in a distribute manner between cages 20 inside the barn 10 via each horizontal heat transfer pipe 300.

That is, in the barn 10, the heat distributor 200, which supplies in a distribute manner the heat source generated at the outside thereof, may be preferably located within a distance of 3 meters from the front of the barn 10, that is, within 3% of a length of the barn 10. This is because when the heat distributor 200 is located in excess of 3%, the optimization of heat distribution becomes difficult.

The temperature of the heat distributor 200 itself is generally about 150 degrees C. A temperature at an outlet of the heat distributor 200, which is connected to the horizontal heat transfer pipe 300, is 60 to 100 degrees C, preferably 60 to 80 degrees C.

The horizontal heat transfer pipe 300, which is connected to the heat distributor 200, is a device that transfers the hot air supplied from the heat distributor 200. The horizontal heat transfer pipe 300 is provided in a space between the cage 20 inside the barn 10 and an outer wall of the barn 10, or between adjacent cages 20. As an example, as illustrated in FIG. 6, the horizontal heat transfer pipe 300, which is connected to the heat source supply part 100 to transfer the hot air supplied from the heat source supply part 100, may be provided in a space between the cage 20 inside the barn 10 and an outer wall of the barn 10, or between adjacent cages 20.

That is, by providing the horizontal heat transfer pipe 300 in the space between the cage 20 and the outer wall of the barn 10 or between adjacent cages 20, it is possible to radiate the hot air toward a floor of the barn 10, air around the horizontal heat transfer pipe 300 and the cages 20 by heat transfer due to convection or conduction.

As an example, when the cage 20 made of metal in the barn has a width of 1 meter in 4 rows and 8 stages, an amount of hot air distributed to one line of the horizontal heat transfer pipe 300 is at a level of approximately 7,000 m³/h. Preferably, the hot air may be distributed to each of five lines.

Further, the length of the horizontal heat transfer pipe 300 may be adjusted according to a position of the heat source supply part 100 disposed inside the barn 10 in consideration of safety. That is, when the heat source supply part 100 is located near an outlet where the cage 20 of the barn 10 is not provided, the length of the horizontal heat transfer pipe 300 may be lengthen, while when the heat source supply part 100 is located near the cage 20, the length of the horizontal heat transfer pipe 300 may be shorten.

The length of the horizontal heat transfer pipe 300 may be preferably 50 % to 98 % of the length of the barn. For example, when a length of a barn to be applied to the present disclosure is 102 meters, the length of the horizontal heat transfer pipe 300 may be preferably 51 to 100 mm (millimeter). A temperature of initial air supplied from the heat source supply part 100 to the horizontal heat transfer pipe 300 may be preferably 50 to 70 degrees C, and a temperature of the hot air at the end of the horizontal heat transfer pipe 300 may be preferably 45 to 60 degrees C.

That is, the temperature of the initial air passing through the horizontal heat transfer pipe 300 falls within the range of 50 to 70 degrees C, and the hot source starting from the horizontal heat transfer pipe 300 is quickly extinguished due to the floor and surroundings of the horizontal heat transfer pipe 300. Further, when the heat source is transferred by a distance of approximately 90 m from the barn 10 in a front-back direction of the barn 10, the final temperature of the hot heat source is in a range of 45 to 60 degrees C. The heat extinguished during such a heat transfer course increases the temperatures of the floor and surroundings of the horizontal heat transfer pipe 300, and the temperature of the cage 20, which makes it possible to enhance the effect of the disinfection.

Further, the horizontal heat transfer pipe 300 may be constituted with a cloth or vinyl to be easily provided between the cage 20 inside the barn 10 and the outer wall of the barn 10, or the floor between adjacent cages 20 in the barn 10.

As illustrated in FIGS. 7 and 8, the horizontal heat transfer pipe 300 may be connected to or provided to be spaced apart from the heat source supply part 100 or the heat distributer 200.

In a case where the horizontal heat transfer pipe 300 is not directly coupled to the heat source supply part 100 or the heat distributer 200 as illustrated in FIG. 8, the blower fan F may be provided inside the horizontal heat transfer pipe 300 to be able to transfer the hot air supplied from the heat source supply part 100 or the heat distributer 200.

The vertical heat blower pipe 400 is provided in the vertical direction inside the barn 10 such that high-temperature heat existing near the ceiling is moved downward.

In addition, the vertical heat blower pipe 400 is provided in the form of a flexible crease pipe having a length of approximately 300 to 400 mm. In a case where the barn has not a layered structure, the vertical heat blower pipe 400 may be provided between adjacent cages 20. In a case where the barn has a layered structure, the vertical heat blower pipe 400 may be provided in an internal space of the cage 20 such that the vertical heat blower pipe 400 extends to the vicinity of the floor and subsequently extends to the space between adjacent cages 20 in the vicinity of the floor. In this case, a plurality of vertical heat blower pipes may be provided in the vertical direction at locations spaced apart by a distance of approximately 2 to 5 meters from the front of the entrance of the barn 10.

Further, the vertical heat blower pipe 400 may be provided in close contact with the cage 20 as needed.

An upper end portion of the vertical heat blower pipe 400 may be formed to be higher than an upper end of the cage 20. Preferably, the vertical heat blower pipe 400 may be located in a range of 1 meter from a position of the support structure 11, which corresponds to a height of the barn 10 except for the roof. That is, when the upper end portion of the vertical heat blower pipe 400 is located at a position below the support structure 11 at 85% or more of the height from the floor of the barn 10 to the support structure 11, the vertical heat blower pipe 400 can easily suck hot air of a large area therearound by virtue of a vortex effect. Further, the upper end portion of the vertical heat blower pipe 400 may be located at a position spaced apart by a distance of approximately 1.5 to 3 meters from the floor of the barn 10.

The blower fan F may be provided inside the vertical heat blower pipe 400 to blow air near the ceiling downward. The blower fan F may be easily provided at a lower end portion of the vertical heat blower pipe 400.

As illustrated in FIG. 9, preferably, at least one or more vertical heat blower pipes 400 may be provided at a position spaced apart by a distance of approximately 1 to 4 meters from the end of the rear portion opposite to the entrance of the barn 10.

A positive pressure of the vertical heat blower pipe 400 is properly in a range of 20 to 80 mmAq, and a blowing amount thereof is preferably 6,000 to 8,000 m³/h per one. That is, the blowing amount of one vertical heat blower pipe 400 may be preferably a level of 0.68 to 0.9 times the total volume of the barn. Further, two vertical heat blower pipes 400 may be preferably provided for a row of one cage. Therefore, an amount of upper heated air blown downward by the vertical heat blower pipe 400 may be preferably in a level of 5 .4 to 7 .2 times the total volume of the barn 10 per hour.

Further, as illustrated FIGS. 7 and 8, the vertical heat blower pipe 400 may be connected to or provided to be spaced apart from the horizontal heat transfer pipe 300 according to the blowing amount.

Further, as illustrated in FIGS. 3 and 9, the vertical heat blower pipe 400 may be connected to the support structure 11 located above the cage 20 with a connection string 30 such as a wire.

As illustrated in FIG. 10, the vertical heat blower pipes 400, which is provided in the vertical direction, may extend to the floor through a space between adjacent cages 20 and be directly connected to the rear end so that both a vertical heat transfer and a horizontal heat transfer can be implemented.

The control part 500 may be connected to the heat source supply part 100 to control the operation of the heat source supply part 100. Further, the control part 500 may be connected to the air circulation pipe 110, the heat distributer 200, the horizontal heat transfer pipe 300, and the vertical heat blower pipe 400 to control the blowing amount flowing therethrough.

As illustrated in FIG. 3, at least one or more temperature sensors 600 may be provided in the ceiling of the barn 10. When a temperature of the ceiling is 70 degrees C or more, the operation of the heat source supply part 100 is stopped responsive to a signal transferred from the control part 500 so that no hot air is supplied and merely air circulates through the air circulation pipe 110.

Although not illustrated, before blowing the hot air for disinfection, silica (silicon dioxide) may be sprayed onto the wall or floor of the barn 10 to eliminate mites by a dry phenomenon.

That is, the mites have a habit of moving to cold places such as gaps between concretes, wires and the like cold due to the hot air applied during disinfection. Thus, the spray of the silica (silicon dioxide) onto the wall or floor of the barn 10 prevents the mites from hiding in or moving to the gaps, which makes it possible to eliminate the mites.

An operation status of the hot-air disinfection system for the poultry barn including the multi-row and multi-level cages of the present disclosure will be described later.

With the operation of the heat source supply part 100, the hot air is transferred between the cage 20 inside the barn 10 and the outer wall of the barn 10 or through each horizontal heat transfer pipe 300 connected between adjacent cages 20. By the hot air transferred through each horizontal heat transfer pipe 300, heat is transferred around each horizontal heat transfer pipe 300 due to the convection or conduction phenomenon so that the heat may be transferred around the floor of the barn 10 and the cages 20.

Further, by spraying and supplying the heat to the plurality of horizontal heat transfer pipes 300 through the use of the heat distributor 200 provided in the front entrance or rear entrance of the livestock ham 10, it is possible to disperse air of a high temperature (in a range of approximately 70 to 90 degrees C) generated in the entrance of the barn 10 and lower the temperature of the air to 60 degrees C or less. This makes it possible to prevent an egg elevator, a feeding device, a chicken-manure transfer device, a water supply device, an electronic equipment, and the like, which are provided on the front side of the barn 10, from exposing to the high temperature, and thus to prevent accidents such as breakage of such components and fires.

On the other hand, when the space on the front side or the rear side of the barn 10 is narrow and consequently the supply of a large-scale heat source and the use of the heat distributor 200 and the like are difficult, at least one or more heat source supply parts 100 may be provided in a passage in which the cage 20 is provided inside the barn 10 to enhance the hot-air efficiency.

In addition, by installing each horizontal heat transfer pipes 300 between the cage 20 provided inside the barn 10 and the outer wall of the barn 10 or between adjacent cages 20, it is possible to evenly transfer the hot air passing through each horizontal heat transfer pipe 300 to the entire space of the barn 10.

Further, by installing the vertical heat blower pipe 400 to extend vertically on the floor in the vicinity of the ceiling in the front portion and the rear portion of the barn 10, it is possible to blow downward the hot air of a high temperature in the vicinity of the ceiling along with the rotation of the blower fan F provided inside the vertical heat blower pipe 400 and lower the high temperature in the vicinity of the ceiling.

In addition, by installing the vertical heat blower pipe 400 to extend vertically on the floor in the vicinity of the ceiling of the barn 10 such that the vertical heat blower pipe 400 extends to the floor and is directly connected to the rear end, it is possible to implement both the vertical heat transfer and the horizontal heat transfer with the vertical heat blower pipe 400 without providing the horizontal heat transfer pipe 300.

As described above, the heat source, which is supplied from the heat source supply part 100 through the horizontal heat transfer pipe 300 respectively distributed to the heat distributor 200, is supplied between the cage 20 inside the barn 10 and the outer wall of the barn 10 or between adjacent cages 20. The high-temperature air in the ceiling is blown downward through the vertical heat blower pipe 400, which makes it possible to minimize a difference in temperature between the lower portion and the upper portion (the vicinity of the ceiling) of the barn 10 and to enhance the disinfection efficiency inside the barn 10.

Although various example embodiments have been described in the above, the present disclosure is not limited to such various specific preferable example embodiments described above, and various modifications may be made by a person of ordinary skill in the art without departing from the scope and technical spirit of the present disclosure.

## Claims

1. A hot-air disinfection system for sterilizing and eliminating bacteria, viruses or pests in a poultry barn with heat, the hot-air disinfection system comprising:
a heat source supply part configured to supply a hot air to an interior of a poultry barn having multi-row and multi-level cages;
a plurality of horizontal heat transfer pipes which are connected to or provided respectively between an outer wall of the poultry barn and the multi-row and multi-level cage or between the multi-row and multi-level cages while being spaced apart from the heat source supply part so that the hot air supplied from the heat source supply part is transferred to a floor of the poultry barn, ambient air and the multi-row and multi-level cages, wherein the plurality of horizontal heat transfer pipes, both ends of which are open, are configured to transfer the hot air;
a vertical heat blower pipe, both ends of which are open, provided vertically in the interior of the poultry barn and configured to blow the hot air near a ceiling of the poultry barn toward the floor by a blower fan provided inside the vertical heat blower pipe; and
a control part configured to control an operation of the heat source supply part.

2. The hot-air disinfection system of Claim 1, wherein the heat source supply part is provided outside the poultry barn and connected to a heat distributer via a front entrance or a rear entrance of the poultry barn including a feed bin and an air suction window so as to supply the hot air to the heat distributer.

3. The hot-air disinfection system of Claim 2, further comprising: an air circulation pipe connected between the heat source supply part and the poultry barn and configured to circulate air in the interior of the poultry barn.

4. The hot-air disinfection system of Claim 3, wherein the air circulation pipe includes a filter configured to filter floating matters in the interior of the poultry barn.

5. The hot-air disinfection system of Claim 1, wherein the heat source supply part includes at least one or more heat source supply parts provided in passages between the multi-row and multi-level cages in the interior of the poultry barn.

6. The hot-air disinfection system of Claim 1, wherein a flow rate of the hot air supplied from the heat source supply part is 5.5 to 7.2 times a total volume of the poultry barn per hour.

7. The hot-air disinfection system of Claim 2, wherein the heat distributor distributes and supplies the hot air supplied from the heat source supply part to each of the plurality of horizontal heat transfer pipes, and a temperature of air at an outlet connected to each of the plurality of horizontal heat transfer pipes is in a range of 60 to 80 degrees C.

8. The hot-air disinfection system of Claim 1, wherein the plurality of horizontal heat transfer pipe are connected to the heat source supply part or the heat distributor and provided respectively between the outer wall of the poultry barn and the multi-row multilayered cages or spaces between the multi-row and multi-level cages, so that the hot air supplied from the heat source supply part is transferred to the floor of the poultry barn, air around the plurality of horizontal heat transfer pipe and the multi-row multi-layered cages.

9. The hot-air disinfection system of Claim 8, wherein, when the plurality of horizontal heat transfer pipes are not directly connected to the heat source supply part or the heat distributer, the blower fan is provided inside each of the plurality of horizontal heat transfer pipes to transfer the hot air.

10. The hot-air disinfection system of Claim 1, wherein a temperature of the hot air supplied from the heat source supply part to the plurality of horizontal heat transfer pipes in an initial stage is in a range of 50 to 70 degrees C, and a temperature of the hot air at an end of each of the plurality of horizontal heat transfer pipes is in a range of 45 to 60 degrees C.

11. The hot-air disinfection system of Claim 1, wherein a length of each of the plurality of horizontal heat transfer pipes is adjusted according to a position at which the heat source supply part is arranged.

12. The hot-air disinfection system of Claim 1, wherein, in a case where the poultry barn has not a layered structure, the vertical heat blower pipe is provided between the multi-row and multi-level cages, while in a case where the poultry barn has not a layered structure, the vertical heat blower pipe is provided in an internal space of each of the multi-row and multi-level cages such that the vertical heat blower pipe extends near the floor and comes out of a space between the multi-row and multi-level cages near the floor so as to blow the hot air around the ceiling downward.

13. The hot-air disinfection system of Claim 12, wherein an upper end portion of the vertical heat blower pipe is located higher than an upper end of each of the multi-row and multi-level cages and provided below a support structure at a position of at least 85% or higher of a height from the floor of the poultry barn to the support structure.

14. The hot-air disinfection system of Claim 12, wherein an upper end portion of the vertical heat blower pipe is located at a place spaced apart by a distance of 1.5 to 3 meters from the floor of the poultry barn.

15. The hot-air disinfection system of Claim 1, wherein the vertical heat blower pipe includes at least one or more vertical heat blower pipes provided in a rear portion opposite an entrance of the poultry barn.

16. The hot-air disinfection system of Claim 1, wherein the vertical heat blower pipe, which is provided vertically in the poultry barn, extends to the floor of the poultry barn and is directly connected to a rear end of the poultry barn so as to implement both a vertical heat transfer and a horizontal heat transfer.

17. The hot-air disinfection system of Claim 1, wherein the vertical heat blower pipe is connected to or provided spaced apart from the plurality of horizontal heat transfer pipes according to a blowing amount.

18. The hot-air disinfection system of Claim 1, wherein a temperature sensor is provided in the ceiling of the poultry barn, and
wherein, when a temperature of the ceiling is 70 degrees C or higher, the operation of the heat source supply part is stopped to circulate air in an air circulation pipe under the control of the control part.

19. The hot-air disinfection system of Claim 1, wherein silica (silicon dioxide) is sprayed onto a wall or the floor of the poultry barn to eliminate mites by a dry phenomenon before disinfection using the hot air.
